(19) Europäisches Patentamt
European Patent Office
Office européen des brevets

(11) Publication number: **0 157 151**
**B1**

(12) # EUROPEAN PATENT SPECIFICATION

(45) Date of publication of patent specification: **13.07.88**

(21) Application number: **85101944.8**

(22) Date of filing: **22.02.85**

(51) Int. Cl.⁴: **C 07 D 213/79,**
C 07 D 213/80,
A 61 K 31/455,
C 07 D 213/803

(54) **New process for preparing cis-3,3,5-trimethylcyclohexyl-D,L-alpha-(3-pyridinecarboxy)-phenylacetate.**

(30) Priority: **07.03.84 IT 1993184**

(43) Date of publication of application:
**09.10.85 Bulletin 85/41**

(45) Publication of the grant of the patent:
**13.07.88 Bulletin 88/28**

(84) Designated Contracting States:
**AT BE CH DE FR GB LI LU NL SE**

(56) References cited:
**DE-A-2 461 909**

**HELVETICA CHIMICA ACTA, vol. 61, Fasc. 5, no. 162, 1978, pages 1675-1681, Basel, CH; P.A. STADLER: "Eine einfache Veresterungsmethode im Eintopf-Verfahren"**

(73) Proprietor: **RAVIZZA S.p.A.**
**35, Via Europa**
**I-20053 Muggio' (Milano) (IT)**

(72) Inventor: **Verga, Alberto**
**Via T. da Cazzaniga 9/6**
**Milano (IT)**

(74) Representative: **Gervasi, Gemma, Dr. et al**
**Studio Brevetti e Marchi NOTARBARTOLO & GERVASI 33, Viale Bianca Maria**
**I-20122 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 157 151 B1

**Description**

This invention relates to a new process for preparing cis-3,3,5-trimethylcyclohexyl-D,L-α-(3-pyridine-carboxy)-phenylacetate of formula:

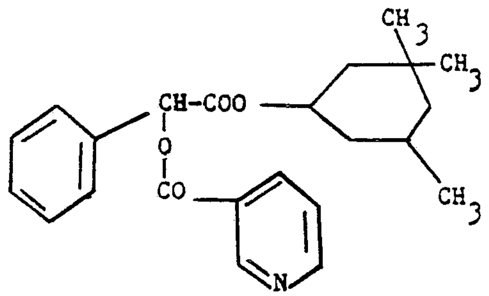

(I)

for use in human therapy as a spasmolytic and vasodilator.

More particularly, the present invention relates to a process for producing cis-3,3,5-trimethylcyclohexyl-D,L-α-(3-pyridine-carboxy)-phenylacetate (I) based on a new reaction cycle which enables the product to be obtained with high yield and high purity characteristics.

The therapeutic activity of cis-3,3,5-trimethylcyclohexyl-D,L-α-(3-pyridine-carboxy)-phenylacetate (I) has been known for some time, as documented for example in European J. Pharmacol. 1, 99—108 (1967); J. Pharm. Pharmac. 19, 667—673 (1967); and J. Pharm. Pharmac. 18, 259—260 (1966).

The excellent properties of this drug have also been confirmed by recent clinical applications. For example, in patients with cerebrovascular insufficiency it has led to an evident psychic and psychoclinical improvement due to improvement in the cortical activity (Archivio di Medicina Interna, XXXV, 4, 295—309 (1983)). In patents affected by chronic arteriopathy in the lower limbs it has led to a significant increase in the free interval (Archivio di Medicina Interna, XXXV, L, 327—332 (1983)).

This drug is also excellently tolerated, and its toxicity is decidedly low.

A process is described in Japanese patent application 22,916/71 for producing cis-3,3,5-trimethylcyclohexyl-D,L-α-(3-pyridine-carboxy)-phenylacetate which consists of reacting 3,3,5-trimethylcyclohexylmandelate with pyridine-3-carboxylic acid or with one of its halides or with its anhydride.

This process presents several difficulties in its practical implementation. Firstly, 3,3,5-trimethylcyclohexylmandelate is a waxy intermediate which contains impurities which are difficult to separate, and therefore does not enable the final product to be obtained with the required purity characteristics or with good yield. Moreover, the reaction must be effected in pyridine, and this represents a serious health hazard. Finally, to effect the reaction it is necessary to apply heat in order to heat the reaction mixture from 100°C to its reflux temperature.

Because of said drawbacks, this process has not found industrial application up to the present time.

We have now discovered that these drawbacks are overcome by the process according to the present invention.

In particular, when operating by the process of the present invention, the intermediate products have crystalline characteristics which enable the final product to be obtained with high purity and high yield, and in the final reaction no pyridine is used, nor is heat supplied. These aspects of the process according to the present invention make it advantageously applicable on an industrial scale.

The process according to the present invention is characterised in that the cis-3,3,5-trimethylcyclohexyl-D,L-α-(3-pyridine-carboxy)-phenylacetate (I) is prepared by the following reaction cycle: pyridine-3-carboxylic acid (II) is reacted with KOH to obtain its potassium salt (III); the product (III) is reacted with oxalyl chloride (IV) to obtain the anhydride of pyridine-3-carboxylic acid (V); the product (V) is reacted with D,L-mandelic acid (VI) to obtain D,L-α-(3-pyridinecarboxy)-phenylacetic acid (VII) which precipitates in the form of the hydrochloride (VIII) when treated with HCl; by treating the product (VIII) with cis-3,3,5-trimethylcyclohexanol (IX), cis-3,3,5-trimethylcyclohexyl-D,L-α-(3-pyridine-carboxy)-phenylacetate (I) is obtained, which is purified in order to give the product at high purity; said purification is conducted by precipitating the cis-3,3,5-trimethylcyclohexyl-D,L-α-(3-pyridine-carboxy)-phenylacetate with p-toluenesulphonic acid, and then freeing it of its p-toluene sulphonate by treatment with an alkaline base.

These and further characteristics of the process according to the present invention, and the relative advantages, are illustrated by the description given hereinafter, which relates to a preferred manner of implementing the invention, given by way of non-limiting examples.

The starting substance used for producing the cis-3,3,5-trimethylcyclohexyl-D,L-α-(3-pyridinecarboxy)-phenylacetate (I) according to the present invention is pyridine-3-carboxylic acid (II), which is reacted with KOH to obtain the relative potassium salt (III) in accordance with reaction (1) and by the following operating procedure:

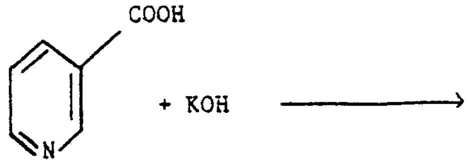


(II) (III) (1)

The potassium hydroxide is dissolved in methyl alcohol in a ratio of between 1:15 and 1:20 by agitation and maintaining the temperature around 35°C. When dissolving is complete, the acid (II) is added in a molar ratio of between 1:1 and 1:1.1 with respect to the potassium hydroxide, and the salt (III) is recovered with a yield of about 95% by partially evaporating the solvent.

The pyridine-3-carboxylic acid potassium salt (III) is reacted with oxalyl chloride (IV) to obtain pyridine-3-carboxylic anhydride (V) in accordance with reaction (2) and by the following operating procedure:

(III) +

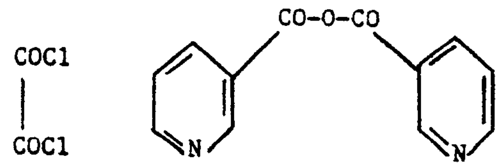

(IV) (V) (2)

The salt (III) is suspended in benzene in a ratio of between 1:3 and 1:5, the suspension is agitated and cooled to a temperature of about 20°C, and a solution of oxalyl chloride in benzene in a ratio of between 1:2 and 1:3 is added dropwise such that the molar ratio of product (III) to product (IV) is between 2:1 and 2:1.2.

After this addition, the mixture is maintained at ambient temperature for a period of between 20 and 30 hours, and is then heated under reflux to boiling for a period preferably of between 1 and 2 hours.

It is filtered while hot, the organic solution is concentrated, and is then cooled to a temperature of about 20°C to obtain product (V) with a yield of about 85%.

The product (V) is treated with D,L-mandelic acid (VI) in accordance with reaction (3) by the following procedure to obtain D,L-α-(3-pyridinecarboxy)-phenylacetic acid (VII):

(V) +

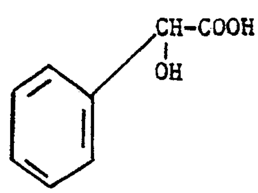

(VI)

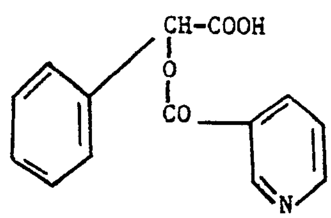

(VII) (3)

The anhydride (V) and acid (VI), in a molar ratio preferably of between 1:1.1 and 1:1.2, are fed into an agitated reactor containing dioxane to an extent such that the ratio of dioxane to the reagents is between 5:1 and 7:1.

The mixture is heated to a temperature of between 70° and 80°C for a time of between 8 and 15 hours. It is cooled to around 25°C, the pyridine-3-carboxylic acid released by the reaction is filtered off, and is dried under vacuum to obtain product (VII).

The product (VII) is dissolved in acetone and is then reprecipitated by treatment with HCl, in the form of the hydrochloride of D,L-α-(3-pyridinecarboxy)-phenylacetic acid (VIII):

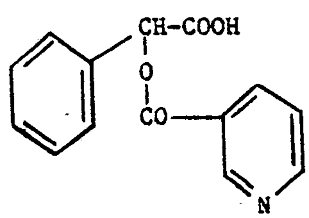

.HCl (VIII)

The hydrochloride (VIII) is preferably obtained by operating under the following conditions: ratio of product (VII) to acetone between 1:2 and 1:3, molar ratio of product (VII) to HCl between 1:1 and 1:1.2, and temperature between 20° and 30°C. The HCl is used as a solution in ethyl alcohol at a concentration of between 15% and 20%.

The product (VIII) is a crystalline product which can be obtained at high purity. The overall yield in passing from the product (V) to the product (VIII) is around 70%.

The product (VIII) is treated with cis-3,3,5-trimethylcyclohexanol (IX) in accordance with reaction (4) and by the following procedure to obtain cis-3,3,5-trimethylcyclohexyl-D,L-α-(3-pyridine-carboxy)-phenylacetate (I):

(VIII) + 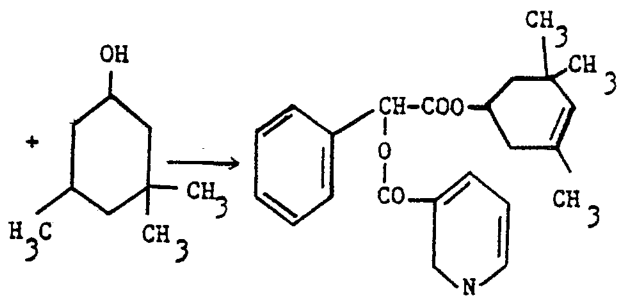 (4)

(IX) (I)

The product (VIII) is added to a suspension of chloromethylene dimethylammoniumchloride in methylene chloride with a ratio of product (VIII) to said suspension of between 1:10 and 1:15, the ratio of the chloromethylene dimethylammoniumchloride to the methylene chloride in said suspension being between 1:15 and 1:25.

The mixture is left to stand for a time of between 12 and 18 hours.

The cis-3,3,5-trimethylcyclohexanol (IX) is dissolved in methylene chloride at a ratio of between 1:2 and 1:3. The molar ratio of product (VIII) to product (IX) is between 1:1.1 and 1:1.2.

The solution containing the product (IX) is added dropwise to the suspension of product (VIII), and the mixture kept under agitation at a temperature of between 20° and 30°C for a period of 5—6 hours.

A volume of water equal to the volume of the reaction mixture is added, and the phases are separated.

The organic phase is evaporated to dryness under reduced pressure, and the crude product is purified by treatment with paratoluene sulphonic acid, by means of which the cis-3,3,5-trimethylcyclohexyl-D,L-α-(3-pyridine-carboxy)-phenylacetate precipitates in the form of paratoluene sulphonate.

The crystalline salt obtained in this manner is treated with an alkaline base in a medium consisting of water and an organic solvent immiscible with water, so as to directly separate the free ester into the organic phase, it being recovered by evaporating the solvent under reduced pressure.

Preferably the paratoluene sulphonate is precipitated in acetone whereas the elimination of the paratoluenesulphonic acid radical is preferably effected with sodium carbonate in a medium consisting of a water-methylene chloride mixture.

The product (I) is obtained in this manner in crystalline form at high purity, with a yield of about 85%.

As stated, all the aforesaid operating conditions are preferred but not critical.

However, one particular embodiment is described hereinafter for the purposes of reproducing the process.

## Example 1

2.55 kg (45 moles) of potassium hydroxide are added to 45 l of methyl alcohol, the mixture being kept under agitation at a temperature of 35°C.

When all the potassium hydroxide has dissolved, 5.55 kg (45 moles) of pyridine-3-carboxylic acid (II) are added over a period of 45 minutes. The mixture is allowed to react for 30 minutes, and after removing 20 l of solvent by evaporation under vacuum, 6.9 kg of pyridine-3-carboxylic acid potassium salt (III) (M.P. 300°C) are recovered with a yield of 95%.

3.22 kg (20 moles) of the salt (III) are suspended in 12.5 l of anhydrous benzene, and 1.27 kg (10 moles) of oxalyl chloride and 3 l of benzene are added over a period of 60 minutes to this suspension, which is kept under agitation at a temperature of 25°C.

When the addition is complete, the mixture is kept for 2 hours at ambient temperature and is then heated under reflux to boiling for 1 hour.

It is filtered while hot, the organic solution is concentrated and is cooled to obtain 1.94 kg of pyridine-3-carboxylic anhydride (V) (M.P. 119—121°C), which is recovered by filtration through a Buchner funnel. The yield is 85%.

3.6 kg (16.8 moles) of the product (V) and 2.6 kg (17.1 moles) of D,L-mandelic acid (VI) are added to 35 l of anhydrous dioxane under agitation.

The mixture is heated to 80°C for 10 hours and is then cooled to ambient temperature, the pyridine-3-carboxylic acid released by the reaction is filtered off, and is evaporated to dryness under vacuum.

In this manner D,L-α-(3-pyridinecarboxy)-phenylacetic acid (VII) is obtained, and is then dissolved in 10 l of acetone and treated with 2.75 l of a 20% w/v solution of hydrochloric acid in ethanol, to obtain a precipitate in the form of the hydrochloride of D,L-α-(3-pyridinecarboxy)-phenylacetic acid (VIII) (M.P. 213—214°C) in a quantity of 3.25 kg, with a yield of 70%.

2.4 kg (8.15 moles) of the product (VIII) are added to a suspension of chloromethylene dimethylammoniumchloride (prepared from 0.9 kg of N,N-dimethylformamide and 1.8 l of thionyl chloride) in 30 l of methylene chloride.

The mixture formed is left standing at ambient temperature for 16 hours to enable the complex to form quantitatively.

A solution consisting of 1.4 kg (10 moles) of cis-3,3,5-trimethylcyclohexanol (IX) in 3.5 l of methylene chloride is added over a period of 45 minutes to said mixture, which is kept under agitation. The mixture is kept under agitation for 5 hours at ambient temperature and is then fed with an equal volume of water, the phases are separated, and the organic phase is evaporated to dryness to obtain the product (I) in its crude state, it then being purified in the following manner.

The crude product is dissolved in 4 l of acetone, to which a solution consisting of 1.5 kg of p.toluenesulphonic acid in 2 l of acetone is then added.

The p.toluene sulphonate thus precipitates in crystalline form, and is then recovered by centrifuging and washed with acetone. This salt is poured into an agitated mixture of 0.8 kg of sodium carbonate in 8 l of water and 4 kg of methylene chloride, and ambient temperature is maintained for 30 minutes.

The aqueous layer is then removed, and the organic layer is washed firstly with a dilute sodium carbonate solution and then with water. The solvent is evaporated at 60°C under a pressure of 1 mm Hg or 133,3 Pa, and 2.6 kg of cis-3,3,5-trimethylcyclohexyl-D,L-α-(3-pyridine-carboxy)-phenylacetate (I) are recovered with high purity, at a yield of 85%.

The following examinations were carried out on the product obtained:

| — Elementary analysis: | C | H | N |
|---|---|---|---|
| calculated: | 72.41 | 7.13 | 3.67 |
| found: | 72.54 | 7.15 | 3.58 |

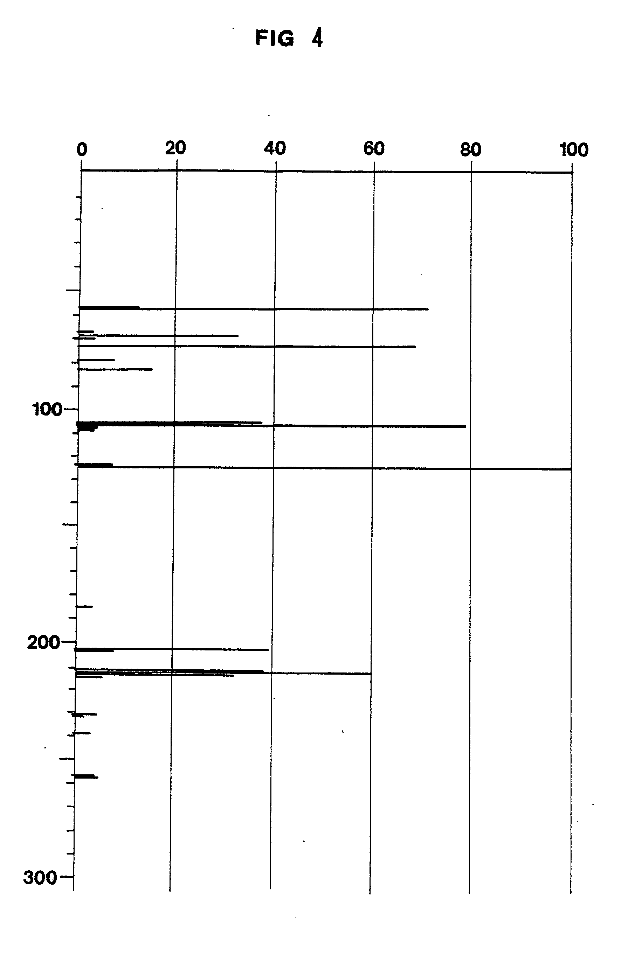

— Melting point: 64—65°C
— I.R. spectrum: Figure 1
— U.V. spectrum: Figure 2
— NMR spectrum: Figure 3
— Mass spectrum: Figure 4

**Claims**

1. A process for producing cis-3,3,5-trimethylcyclohexyl-D,L-α-(3-pyridinecarboxy)-phenylacetate (I), characterized in that pyridine-3-carboxylic acid (II) is reacted with a methanolic solution of KOH to obtain its potassium salt (III);

(III) is reacted with oxalyl chloride (IV) to obtain the anhydride of pyridine-3-carboxylic acid (V);

(V) is reacted with D,L-mandelic acid (VI) to obtain D,L-α-(3-pyridinecarboxy)-phenylacetic acid (VII) which precipitates in the form of the hydrochloride (VIII) when treated with HCl;

(VIII) is added to a suspension of chloromethylene dimethylammoniumchloride in methylene chloride and the suspension is then reacted with cis-3,3,5-trimethylcyclohexanol (IX) dissolved in methylene chloride to obtain crude (I), which is furtherly purified by precipitating with p-toluenesulphonic acid in acetone and then freeing from by treatment with an alkaline base.

2. A process as claimed in claim 1, wherein the reaction of (III) with (IV) is run in benzene, the molar ratio of (III) to (IV) being of from 2:1 to 2:1.2, at room temperature for a time of from 20 to 30 hours and then at reflux temperature for a time of from 1 to 2 hours.

3. A process as claimed in claim 1, wherein (V) is reacted with (VI) in dioxane as reaction medium, the molar ratio of (V) to (VI) being between 1:1.1 and 1:1.2, at a temperature of from 70° to 80°C for a time of from 8 to 15 hours.

4. A process as claimed in 1, wherein the methylene chloride solution of (IX) is added dropwise to the said suspension of (VIII), the mixture being then maintained at a temperature of from 20° to 30°C for a time of from 5 to 6 hours, the molar ratio of (VIII) to (IX) being of from 1:1.1 and 1:1.2.

5. A process as claimed in 1, wherein the precipitate of (I) with p-toluenesulfonic acid is treated with sodium carbonate in a medium consisting of a mixture of water and methylene chloride, the organic and water phase are then separated and pure (I) is recovered by evaporating the organic phase under reduced pressure.

**Patentansprüche**

1. Verfahren zum Herstellen von cis-3,3,5-Trimethylcyclohexyl-D,L-α-(3-pyridincarboxy)-phenylacetat (I), dadurch gekennzeichnet, daß Pyridin-3-carbonsäure (II) mit einer methanolischen KOH-Lösung umgesetzt wird, wobei deren Kaliumsalz (III) erhalten wird; (III) mit Oxalylchlorid (IV) umgesetzt wird, wobei das Anhydrid (V) der Pyridin-3-carbonsäure erhalten wird; (V) mit D,L-Mandelsäure (VI) umgesetzt wird, wobei die D,L-α-(3-Pyridincarboxy)-phenylessigsäure (VII) erhalten wird, die bei Behandlung mit HCl in Form des Hydrochlorids (VIII) ausfällt; (VIII) zu einer Suspension von Chlormethylendimethylammoniumchlorid in Methylenchlorid zugesetzt und die Suspension dann mit cis-3,3,5-Trimethylcyclohexanol (IX), gelöst in Methylenchlorid, umgesetzt wird, wobei das rohe Produkt (I) erhalten wird, das dann durch Ausfällen mit p-Toluolsulfonsäure in Aceton gereinigt und durch Behandlung mit einer alkalischen Base freigesetzt wird.

2. Verfahren, wie in Anspruch 1 beansprucht, worin die Reaktion von (III) mit (IV) in Benzol bei einem Molverhältnis von (III) zu (IV) von 2:1 bis 2:1,2 bei Raumtemperatur während eines Zeitraumes von 20 bis 30 Stunden und dann bei Rückflußtemperatur während eines Zeitraumes von 1 bis 2 Stunden durchgeführt wird.

3. Verfahren, wie in Anspruch 1 beansprucht, worin (V) mit (VI) in Dioxan als Reaktionsmedium mit einem Molverhältnis von (V) zu (VI) von 1:1,1 bis 1:1,2 bei einer Temperatur von 70 bis 80°C während eines Zeitraumes von 8 bis 15 Stunden durchgeführt wird.

4. Verfahren, wie in Anspruch 1 beansprucht, worin die Methylenchloridlösung von (IX) tropfenweise zu der genannten Suspension von (VIII) zugesetzt wird, die Mischung dann während eines Zeitraumes von 5 bis 6 Stunden bei einer Temperatur von 20 bis 30°C gehalten wird und das Molverhältnis von (VIII) zu (IX) 1:1,1 bis 1:1,2 beträgt.

5. Verfahren, wie in Anspruch 1 beansprucht, worin der Niederschalg von (I) mit p-Toluolsulfonsäure mit Natriumcarbonat in einem Medium bestehend aus einer Mischung von Wasser und Methylenchlorid behandelt wird, die organische und die Wasserphase dann getrennt werden und reines (I) durch Eindampfen der organischen Phase unter vermindertem Druck gewonnen wird.

**Revendications**

1. Procédé de préparation du D,L-α-(3-pyridinecarboxy)-phényl-acétate de cis-3,3,5-triméthyl-cyclohexyle (I), caractérisé par le fait qu'on fait réagir l'acide pyridine-3-carboxylique (II) avec une solution méthanolique de KOH pour obtenir son sel de potassium (III);

on fait réagir (III) avec du chlorure d'oxalyle (IV) pour obtenir l'anhydride de l'acide pyridine-3-carboxylique (V);

on fait réagir (V) avec l'acide D,L-α-mandélique (VI), pour obtenir l'acide D,L-α-(3-pyridine-carboxy)-phénylacétique (VII) qui précipite sous la forme du chlorhydrate (VIII) lorsqu'il est traité par HCl;

on ajoute (VIII) à une suspension de chlorure de chlorométhylènediméthylammonium dans du chlorure de méthylène, puis on fait réagir la suspension avec udu cis-3,3,5-triméthyl-cyclohexanol (IX) dissous dans du chlorure de méthylène pour obtenir le produit (I) brut, qui est soumis à une purification supplémentaire par précipitation avec de l'acide p-toluènesulfonique dans de l'acétone, puis libération par traitement avec une base alcaline.

2. Procédé selon la revendication 1, dans lequel la réaction de (III) avec (IV) est effectuée dans du benzène, le rapport molaire de (III) à (IV) étant de 2:1 à 2:1,2, à la température ambiante, pendant une durée de 20 à 30 h, puis à la température du reflux pendant une durée de 1 à 2 h.

3. Procédé selon la revendication 1, dans lequel (V) est mis à réagir avec (VI) dans du dioxane comme milieu réactionnel, le rapport molaire de (V) à (VI) étant compris entre 1:1,1 et 1:1,2, à une température de 70 à 80°C, pendant une durée de 8 à 15 h.

4. Procédé selon la revendication 1, dans lequel la solution de (IX) dans le chlorure de méthylène est ajoutée goutte à goutte à cette suspension de (VIII), le mélange étant ensuite maintenu à une température de 20 à 30°C pendant une durée de 5 à 6 h, le rapport molaire de (VIII) à (IX) étant de 1:1,1 à 1:1,2.

5. Procédé selon la revendiction 1, dans lequel le précipité de (I) avec l'acide p-toluène sulfonique est traité par le carbonate de sodium dans un milieu constitué d'un mélange d'eau et de chlorure de méthylène, la phase organique et la phase aqueuse sont ensuite séparées, et le (I) pur est recueilli en faisant évaporer la phase organique sous pression réduite.

FIG 1

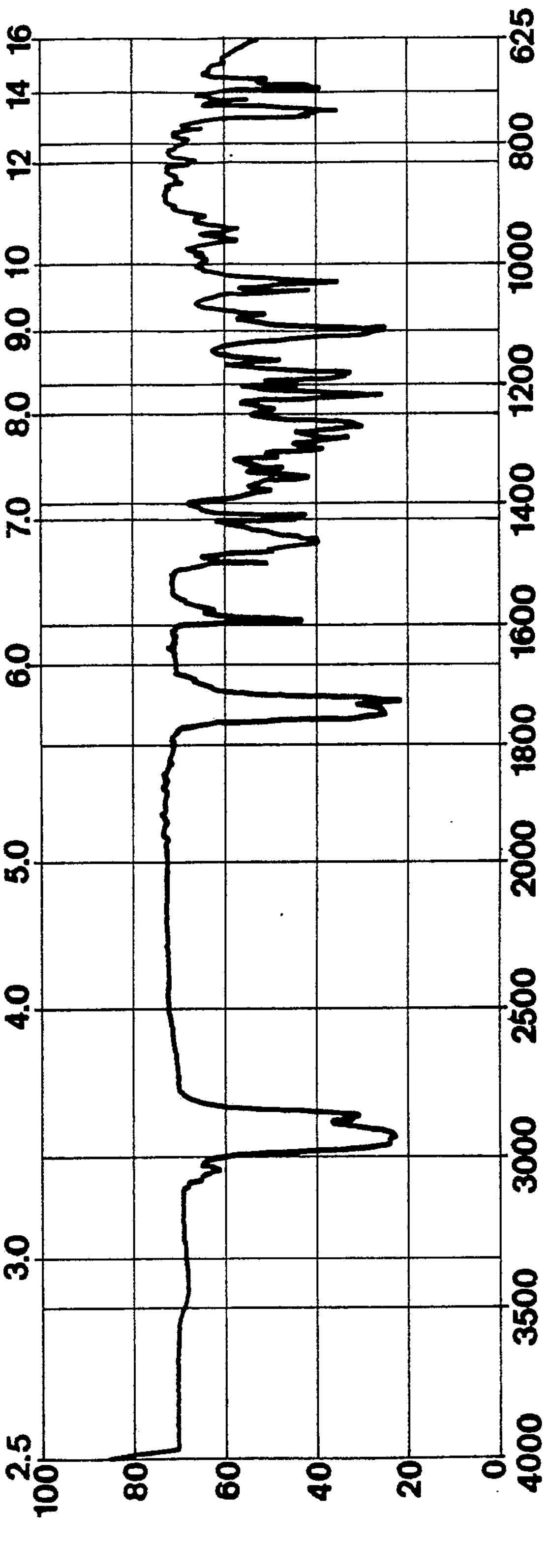
2.5
3.0
4.0
5.0
6.0
7.0
8.0
9.0
10
12
14
16
100
80
60
40
20
0
4000
3500
3000
2500
2000
1800
1600
1400
1200
1000
800
625

FIG 2

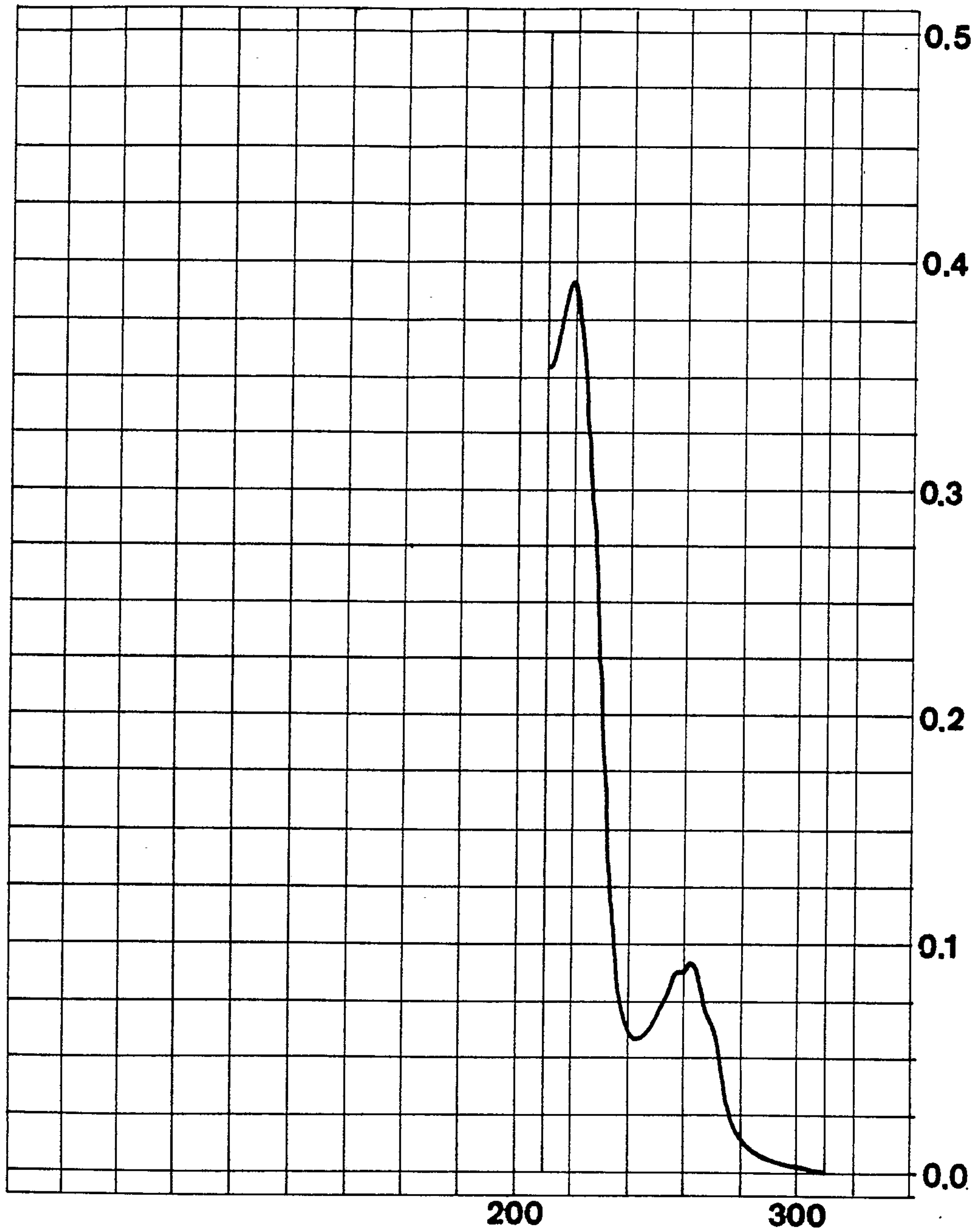

FIG 3

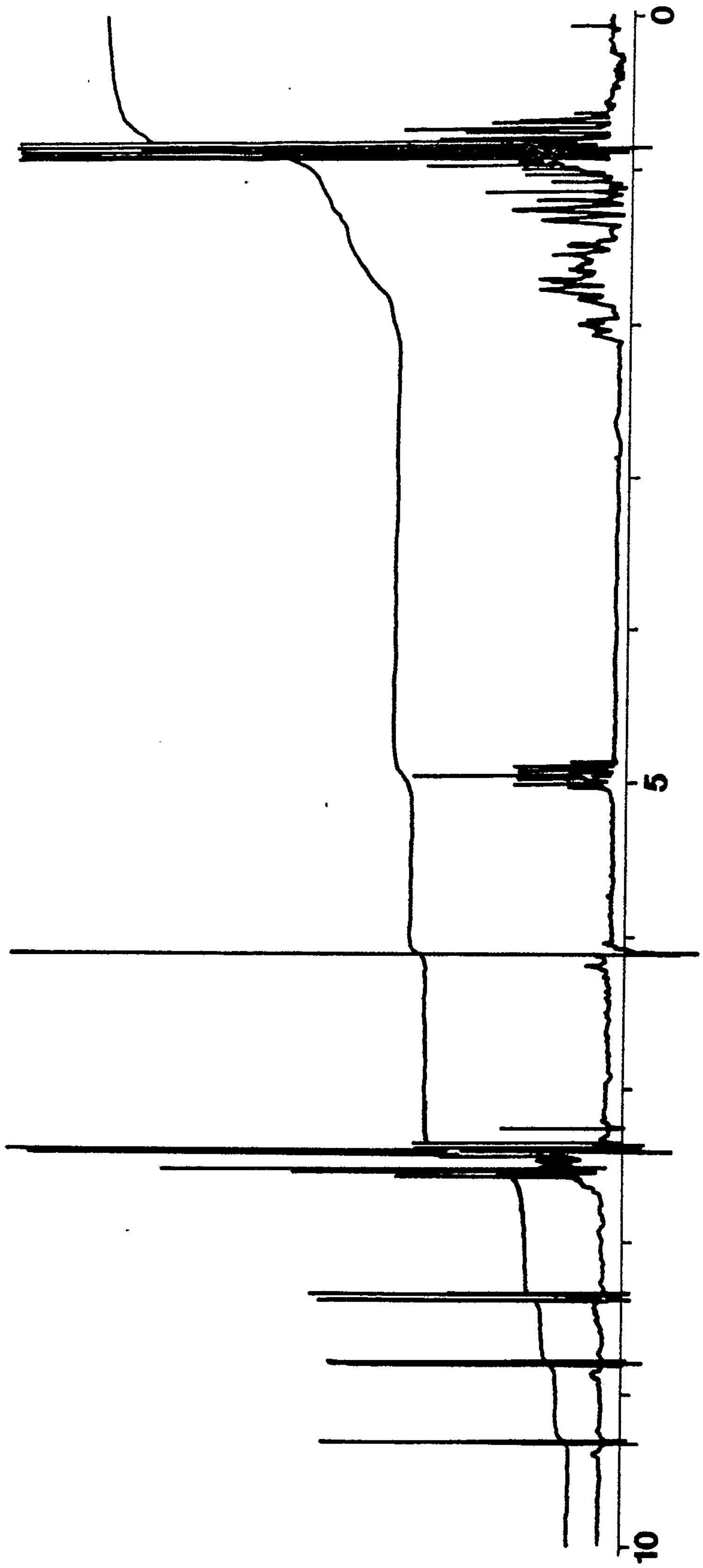

FIG 4